# EUROPEAN PATENT SPECIFICATION

(11) **EP 3 605 053 B1**
(45) Date of publication and mention of the grant of the patent: **04.05.2022**
(21) Application number: 18771829.1
(22) Date of filing: 22.03.2018
(51) Int. Cl.: G01N 1/02, A61B 5/091, G01N 1/00, G01N 33/497, A61B 5/08, A61B 5/097, A61B 5/087, A61B 5/00

(54) **EXHALED AIR MEASUREMENT DEVICE**
VORRICHTUNG ZUR MESSUNG AUSGEATMETER LUFT
DISPOSITIF DE MESURE D'AIR EXPIRÉ

(30) Priority: 23.03.2017 JP 2017057132
(43) Date of publication of application: 05.02.2020
(73) Proprietor: PHC Holdings Corporation, Tokyo 105-8433 (JP)
(72) Inventor: OKI Kenji, Ehime 791-0395 (JP); KONISHI Takanori, Ehime 791-0395 (JP); KIMURA Takashi, Ehime 791-0395 (JP)
(74) Representative: Grünecker Patent- und Rechtsanwälte PartG mbB
(86) International application number: PCT/JP2018/011483
(87) International publication number: WO 2018/174182

(56) References cited:
- WO-A1-2015/001735
- JP-A- S56 163 634
- JP-A- 2010 025 718

## Description

### TECHNICAL FIELD

The present disclosure relates to an exhalation measurement device to be used when e.g. detecting asthma or checking pulmonary function.

### BACKGROUND ART

Exhalation measurement devices for detecting a specific gas that is contained in the exhalation of a subject, for use in disease diagnosis or pulmonary function evaluation, have been put into practical use. For example, Patent Document 1 discloses a diagnostic gas analyzer that detects nitrogen monoxide (NO) within exhalation. Patent Document 1 states that the amount of nitrogen monoxide within exhalation can be used as an index of pneumonia diagnosis, or put to clinical use for asthma patients. WO 2015/001735 A1 relates to an exhaled gas measurement device having the features of preamble of claim 1.

### CITATION LIST

### PATENT LITERATURE

Japanese National Phase PCT Laid-Open Publication No. 2005-538819

### SUMMARY OF INVENTION

### TECHNICAL PROBLEM

Since the content of any specific gas, e.g., nitrogen monoxide, within exhalation would be very small, an exhalation measurement device is required to be able to stably detect the amount of specific gas while maintaining high accuracy. The present disclosure provides an exhalation measurement device which is capable of stably detecting the amount of specific gas while maintaining high accuracy.

### SOLUTION TO PROBLEM

An exhalation measurement device according to the invention is defined in claim 1. Further advantageous embodiments are defined in dependent claims.

### ADVANTAGEOUS EFFECTS OF INVENTION

With an exhalation measurement device according to the present disclosure, it is possible to stably detect the amount of specific gas while maintaining high accuracy.

### BRIEF DESCRIPTION OF DRAWINGS

[FIG. **1]** FIG. **1** is a perspective view showing an exemplary embodiment of an exhalation measurement device according to the present disclosure.
[FIG. **2**] FIG. **2** shows an example control block diagram of the exhalation measurement device.
[FIG. **3]** FIG. **3** is a perspective view showing an example of the interior of a main body of the exhalation measurement device.
[FIG. **4]** FIG. **4** is a cross-sectional view showing an example of a handle section.
[FIG. **5A]** FIG. **5A** is a schematic diagram showing an example of an adjuster.
[FIG. **5B]** FIG. **5B** is a schematic diagram showing an example where a gauge pressure sensor is used as a pressure sensor.
[FIG. **5C**] FIG. **5C** is a schematic diagram showing an example where a differential pressure sensor is used as a pressure sensor.
[FIG. **5D]** FIG. **5D** is a schematic diagram showing an example where an atmospheric pressure sensor is used as a pressure sensor.
[FIG. **6]** FIG. **6** is a cross-sectional view showing an example of a reference gas generator.
[FIG. **7**] FIG. **7** is a schematic diagram showing an example of a switching device.
[FIG. **8A**] FIG. **8A** is a schematic diagram showing an example of a flow rate detector.
[FIG. **8B**] FIG. **8B** is a schematic diagram showing an example of a flow rate detector.
[FIG. **9]** FIG. **9** is a flowchart showing an exemplary operation of the exhalation measurement device.
[FIG. **10A**] FIG. **10A** is a graph showing a result of examining the characteristics of reference gas generators.
[FIG. **10B**] FIG. **10B** is a graph showing a result of examining the characteristics of reference gas generators.

### DESCRIPTION OF EMBODIMENTS

The inventors have sought for a method which can stably measure a specific gas content within exhalation while maintaining high accuracy. In order to enhance the accuracy of measurement, the amount of specific gas contained in the inhalation of a subject should preferably be reduced as much as possible in advance. Moreover, a reference gas which was generated under conditions resembling inhalation may be provided, and the specific gas content within exhalation may be measured against this reference gas. In order to generate such a reference gas, the specific gas within the reference gas should also preferably be reduced as much as possible.

In order to reduce the amount of specific gas within air, use of a reference gas generator may be possible, such that the reference gas generator includes a filter which is filled with an adsorbent that adsorbs the specific gas. The inventors have tried producing an exhalation measurement device equipped with such a reference gas generator, and measuring the amount of specific gas within exhalation under various conditions, thereby finding that the reference gas generator may deteriorate early on in some cases. In looking for the causes thereof, they found that at a medical institution, etc., where the exhalation measurement device is used, other gases may exist in the surrounding environment which induce early deterioration of the adsorbent that adsorbs the specific gas. For example, in a medical institution or the like, ethyl alcohol may be used for disinfection of medical equipment, or disinfection of the patient's skin, etc. Hereinafter, ethyl alcohol will simply be referred to as alcohol in the present specification. If any such alcohol exists in the environment in which the exhalation measurement device is stored or in the environment of use, the adsorbent will adsorb the alcohol, and be degraded with respect to its ability to adsorb the specific gas. Especially when the exhalation measurement device is stored in its dedicated case or the like after alcohol disinfection, alcohol may vaporize within the dedicated case, so that a temporarily high concentration of alcohol may exist. In such cases, the high concentration of alcohol may be adsorbed by the adsorbent, thereby significantly deteriorating the adsorbent's ability to adsorb the specific gas.

In view of such problems, the inventors have arrived at a novel exhalation measurement device. In summary, an exhalation measurement device according to the present disclosure is as follows.

Hereinafter, with reference to the drawings, an embodiment of an exhalation measurement device according to the present disclosure will be described. An exhalation measurement device according to the present disclosure may measure the concentration of nitrogen monoxide within exhalation, for example. In the present embodiment, inhalation means the air that is sucked into a subject who breathes in, or the air that moves during a sucking motion of the subject. On the other hand, exhalation means a gaseous body that is discharged out of the subject as the subject breathes out.

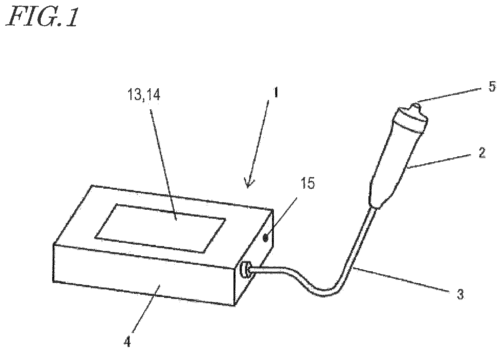

FIG. **1** is a perspective view showing the appearance of an exhalation measurement device **1.** The exhalation measurement device **1** includes, for example, a handle section **2,** a main body **4,** and a handle tube **3** that connects the handle section **2** and the main body **4.**

A subject holds the handle section **2** in a hand, and after breathing out, places an orifice **5** of the handle section **2** against his or her mouth, and breathes in while in this state. Thereafter, as he or she breathes out, exhalation flows from the orifice 5 into the handle section **2,** whereby the exhalation is introduced into the main body **4** via the handle tube **3.** The main body **4** measures the nitrogen monoxide concentration within the exhalation, and displays the result of measurement on a display device **14** of the main body **4.**

### [structure of the exhalation measurement device 1]

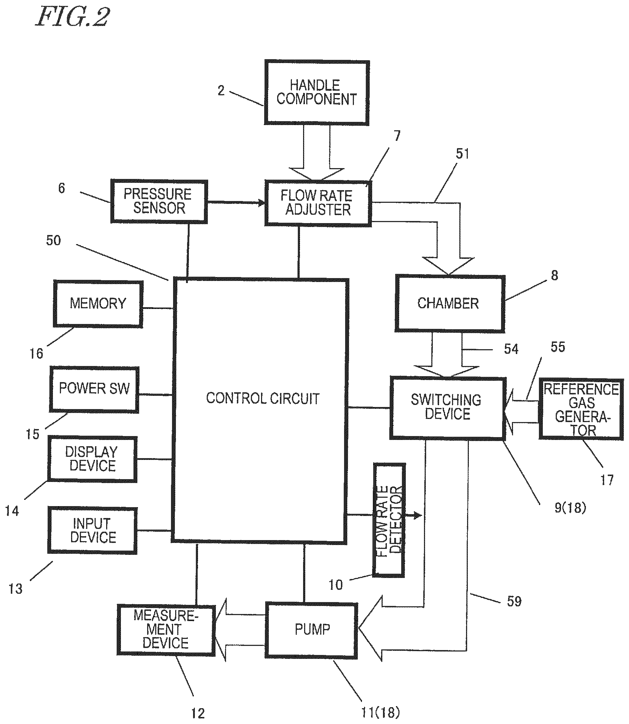

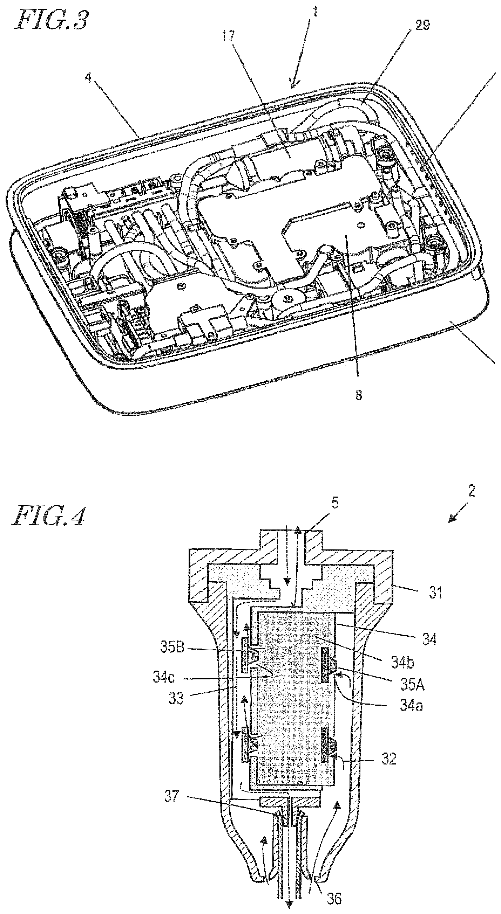

FIG. **2** is a control block diagram of the main body **4.** In FIG. **2****,** thick arrows schematically show channels through which a gas moves, as well as directions of its movement. FIG. **3** is a perspective view showing the interior of the main body **4.** Component elements of the main body **4** are accommodated in a main housing **41.** The main body **4** includes: a pressure sensor **6;** a flow rate adjuster **7;** a chamber **8;** a measurement device **12;** an input device **13;** the display device **14;** a power switch **15;** a memory **16;** a reference gas generator **17;** a gas transporter **18** including a switching device **9,** a flow rate detector **10,** and a pump **11;** and a control circuit (control device) **50.**

The exhalation measurement device **1** is controlled by the control circuit **50.** The control circuit **50** includes electronic parts with processing circuitry for performing information processing, such as a CPU provided in the exhalation measurement device **1;** it is also called a microcomputer. By executing a computer program that is loaded to the memory **16,** the control circuit **50** sends instructions to other component elements, in accordance with the procedure of the computer program. Receiving such instructions, the respective component elements operate as will be described in the present specification. The procedure of the computer program will be described later.

The input device **13** is a device with which a person, e.g., a subject, who operates the exhalation measurement device **1** inputs information to the control circuit **50,** including for example a touch screen panel that is integrally provided within the display device **14.** The power switch **15** is provided in the main housing **41,** for example.

The memory to which the computer program is loaded may be volatile or non-volatile. The volatile memory (RAM) is a RAM that cannot retain its stored information while not being powered. For example, a dynamic random access memory (DRAM) is a typical volatile RAM. A non-volatile RAM is a RAM that is capable of retaining information without being powered. For instance, magnetoresistive RAMs (MRAM), resistive random access memories (ReRAM), and ferroelectric resistive random access memories (FeRAM) are examples of non-volatile RAMs. In the present embodiment, a non-volatile RAM is preferably adopted. A volatile RAM and a non-volatile RAM are both examples of non-transitory and computer-readable storage media. Moreover, a magnetic storage medium such as a hard disk, and an optical storage medium such as an optical disc, are also examples of non-transitory and computer-readable storage media. That is, a computer program according to the present disclosure may be recorded on any of various non-transitory computer-readable media, other than any medium such as the atmospheric air (i.e., a transitory medium), that transmits the computer program as a radio wave signal. Hereinafter, each component element will be described in detail.

### (handle section 2)

The handle section **2** is an exhalation generation unit that causes a subject's exhalation to be generated. In the present embodiment, the exhalation generation unit is accommodated, as the handle section **2,** in a separate housing from the main body **4.** FIG. **4** shows a schematic cross section of an example of the handle section **2.** The handle section **2** includes a sub-housing **31,** an inhalation path **32,** an exhalation path **33,** and a second filter **34.** The sub-housing **31** has an outer shape that allows the subject to grip it, for example. Specifically, the sub-housing **31** may have a substantially cylindrical outer shape that can be gripped with the palm of a hand, with a suction hole **36** and a discharge hole **37** being provided at one end of the cylindrical sub-housing **31,** for example. At the other end of the cylindrical sub-housing **31,** the orifice **5,** through which inhalation and exhalation of the subject pass, is provided. The inhalation path **32** connects between the orifice **5** and the suction hole **36,** such that the second filter **34** is positioned in the inhalation path **32.** The exhalation path **33** connects between the orifice **5** and the discharge hole **37.** The inhalation path **32** and the exhalation path **33** are partitioned by the piece that composes the sub-housing **31** or the like, for example.

As the subject breathes in, while holding against his or her mouth the surface of the sub-housing **31** in which the orifice **5** is made, inhalation (air) is introduced into the inhalation path **32.**

The second filter **34** inserted in the inhalation path 32 has apertures **34a** and **34c** and one-way valves **35A** and **35B.** When the handle section **2** is not being used by the subject, the one-way valves **35A** and **35B** close the apertures **34a** and **34c,** respectively.

As the subject breathes in, the one-way valves **35A** and **35B** open the apertures **34a** and **35c,** respectively, and the inhalation within the inhalation path **32** is introduced through the aperture(s) **34a** into the second filter **34.** The second filter **34,** which contains an adsorbent **34b** that adsorbs a specific gas, adsorbs the specific gas within the inhalation as the inhalation passes through the adsorbent **34b.** In the present embodiment, the specific gas is nitrogen monoxide, so that nitrogen monoxide within the inhalation is adsorbed by the adsorbent **34b.** The inhalation, from which nitrogen monoxide has been removed to a predetermined concentration or less, is discharged out of the second filter **34** through the aperture(s) **34c** equipped with the one-way valve (s) **35,** and is sucked into the body of the subject through the orifice **5.**

As the subject breathes in and then breathes out, his or her exhalation is introduced through the orifice **5** into the exhalation path **33.** In the present embodiment, inhalation and exhalation pass through the orifice **5.** This means that the inhalation path **32** and the exhalation path **33** at least have a common portion. In the present embodiment, the region extending from a region of the inhalation path **32** that is in contact with the one-way valve(s) **35** to the orifice **5** defines a path that is common to the exhalation path **33.** In other words, the exhalation which is introduced into the exhalation path **33** is in contact with the one-way valve(s) **35.** However, owing to a pressure difference between the exhalation path **33** and the interior of the second filter **34,** the one-way valve(s) **35** closes the aperture(s) **34c** so that the exhalation does not flow into the second filter **34.** The exhalation passing through the exhalation path **33** is released out of the handle section **2** through the discharge hole **37.** The discharge hole **37** has the handle tube **3** connected thereto, such that exhalation is introduced into the main body **4** through the handle tube **3.**

Since the handle section **2** constitutes a separate piece from the main body **4,** when the subject carries out an exhalation measurement, he or she may hold only the handle section **2** in order to generate exhalation. Since the subject does not need to hold the main body **4,** the subject's burden during examination is reduced. Moreover, excellent operability is provided because the position of the main body **4** may be relatively freely determined, such that the information that is displayed by the display device **14** of the main body **4** will be easy to see. Moreover, in the present embodiment, nitrogen monoxide is removed from the air (i.e., inhalation) that is sucked by the subject in generating exhalation. By removing as much nitrogen monoxide, which is the target of measurement, from the inhalation as possible, influences of the measurement environment on the measurement can be reduced.

In the event that the second filter **34** deteriorates in terms of adsorption performance, the handle section **2** may be constructed so that the entire handle section **2** can be exchanged, or that only the second filter **34** can be exchanged.

In the present embodiment, the exhalation generation unit is composed of the handle section **2** including the sub-housing **31,** which is separate from the main body **4.** However, for example, the exhalation generation unit may be integral with the main body **4,** or composed as a mouthpiece which is detachably mounted to and supported on the main body **4.** In this case, the subject may carry out measurement by holding against his or her mouth the mouthpiece mounted on the main body **4,** while supporting the main body **4** with a hand.

### (chamber 8)

The chamber **8** has a space for temporarily retaining the exhalation that is generated by the handle section **2.** The exhalation which has been introduced from the handle tube **3** to the main body **4** is transported via an introduction path **51** to the chamber **8,** owing to the pressure of the exhalation that is put out from the subject. The space in the chamber **8** has a sufficient volumetric capacity for measuring the concentration of the specific gas. Prior to the measurement, the chamber **8,** the handle section **2,** the handle tube **3,** and the introduction path **51** are filled with the same air as the external environment. Therefore, the chamber **8** having a discharge port, the air filling these portions is first discharged through the discharge port from the chamber **8** while the subject keeps breathing out for a certain period of time, and thereafter the exhalation fills the chamber **8.**

### (flow rate adjuster 7)

When generating exhalation, the concentration of the specific gas within the exhalation may vary depending on the intensity (pressure) and flow rate with which the subject breathes out. In order to carry out measurement under conditions that are as constant as possible, preferably a structure for adjusting the flow rate of exhalation is provided in the introduction path **51** leading to the chamber **8,** so that exhalation will be generated within a certain range of pressure and flow rate. For this purpose, in the present embodiment, the main body **4** includes the flow rate adjuster **7** and the pressure sensor **6,** such that a pressure in the introduction path **51** is detected by the pressure sensor **6,** and, based on the result of detection, a flow rate of exhalation flowing in the introduction path **51** is adjusted by the flow rate adjuster **7.**

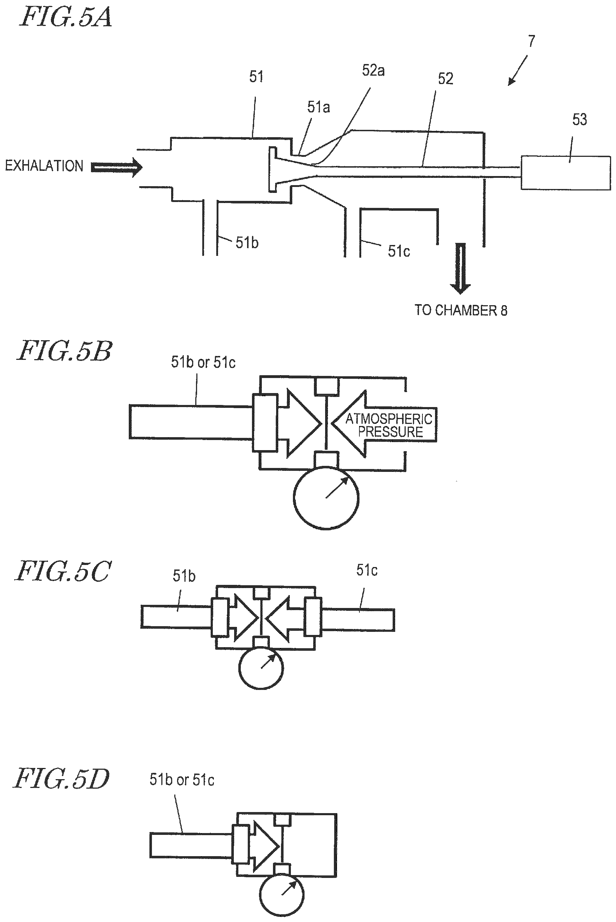

FIG. **5A** is a schematic diagram showing an exemplary structure of the flow rate adjuster **7.** The flow rate adjuster **7** includes an adjustment valve **52** having a tapered portion **52a,** and a driving device **53**. An adjustment hole **51a** is provided midway in the introduction path **51,** and the tapered portion **52a** of the adjustment valve **52** is inserted through the aperture of the adjustment hole **51a** so that, as the driving device **53** moves the adjustment valve **52,** the tapered portion **52a** adjusts the size of the aperture, thereby adjusting the flow rate of the exhalation flowing in the introduction path **51.** The driving device **53** may be a motor, piezoelectric element, or the like. The flow rate adjuster **7** may be a solenoid valve.

### (pressure sensor 6)

The pressure sensor **6** detects a pressure in the introduction path **51.** Various types of pressure sensors can be used for the pressure sensor. The pressure in the introduction path **51** to be detected by the pressure sensor **6** may be found at: two points, i.e., a port **51b** that is located between an inlet through which exhalation is introduced into the main body **4** and the flow rate adjuster **7,** and a port **51c** that is located between the flow rate adjuster **7** and the chamber **8;** or one point, i.e., the port **51b** that is located between the inlet through which exhalation is introduced into the main body **4** and the flow rate adjuster **7.**

Specifically, the pressure sensor **6** may be a gauge pressure sensor, a differential pressure sensor, an atmospheric pressure sensor, or the like. FIG. **5B** schematically shows a construction where pressure is measured by a gauge pressure sensor. The gauge pressure sensor measures a difference in pressure between a space that is connected to the pressure sensor and the atmospheric air. In this case, the pressure sensor **6** may measure a pressure in the introduction path **51** by using the port **51b,** or measure pressures by using the port **51b** and the port **51c.**

FIG. **5C** schematically shows a construction where pressure is measured by a differential pressure sensor. The differential pressure sensor measures a pressure difference between two spaces that are connected to the pressure sensor. In this case, the pressure sensor **6** take a measurement at the port **51b** and the port **51c.**

FIG. **5D** schematically shows a construction where pressure is measured by an atmospheric pressure sensor. The atmospheric pressure sensor measures an atmospheric pressure in a space that is connected to the pressure sensor. In this case, the pressure sensor **6** may measure a pressure in the introduction path **51** by using the port **51b,** or measure pressures by using the port **51b** and the port **51c.**

### (reference gas generator 17)

The reference gas generator **17** generates a reference gas. The reference gas is preferably the air in an environment which is similar to the inhalation to be sucked in by the subject. This allows conditions other than the specific gas for measurement to be as identical as possible, so that the influence of any conditions other than the specific gas that is exerted on the measurement will be reduced as much as possible. More specifically, it is preferable that: the reference gas generator **17** is capable of generating, as a reference gas, the air which is acquired from a surrounding environment of the exhalation measurement device **1** during measurement but from which the specific gas has been removed.

As shown in FIG. **3****,** the reference gas generator **17** is disposed inside the main body **4.** On the side surface of the main body **4** near the reference gas generator **17,** a plurality of external air holes **41a** are provided. Through the external air holes **41a,** external air is taken into the main body **4** of the measurement device, thus allowing the measurement environment (e.g., temperature and humidity) inside the main body **4** of the measurement device to be identical with the external environment.

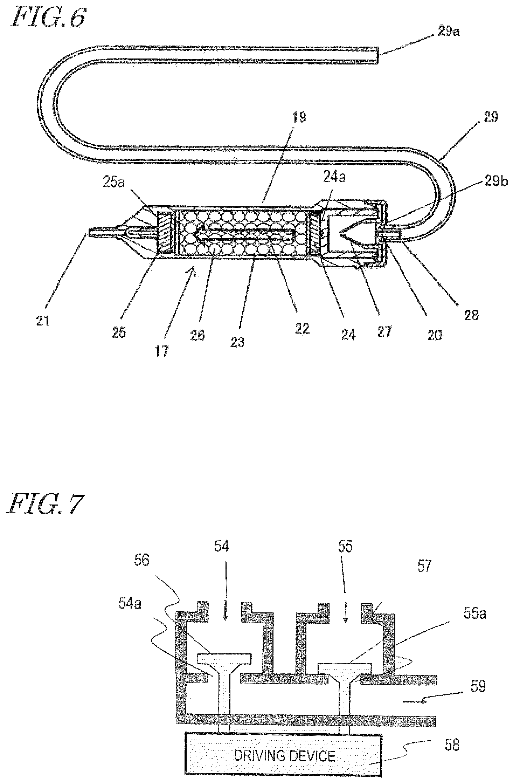

FIG. **6** is a cross-sectional view of the reference gas generator **17.** The reference gas generator **17** includes an elongated cylindrical case **19,** a first filter **23,** and a supply tube **29.** At the two ends of its longitudinal direction, the case **19** has an intake port **20** and an exhaust port **21.** The intake port **20** and the exhaust port **21** are made with a smaller diameter than the inner diameter of the case **19.** The case **19** has an aeration path **22** that connects between the intake port **20** and the exhaust port **21.** Around (on the outer surface of) the intake port **20,** a cylindrical, outwardly-protruding attachment section **28** is provided.

The first filter **23** is disposed in the aeration path **22.** In the present embodiment, the first filter **23** has a cylindrical shape, and includes an air-permeable gasket **24** disposed closer to the intake port **20,** an air-permeable gasket **25** disposed closer to the exhaust port **21,** and an adsorbent **26** that adsorbs a specific gas, which fills between the gasket **24** and the gasket **25.** A surface of the gasket **24** that is on the intake port **20** side will be referred to as a first aperture **24a,** and a surface of the gasket **25** that is on the exhaust port **21** side will be referred to as a second aperture **25a.**

Inside the case **19** and between the intake port **20** and the first aperture **24a** of the gasket **24,** a check valve **27** is provided. The check valve **27** is composed of a pair of thin rubber plates, such that its end toward the gasket **24** is opened and closed. Specifically, when air moves from the intake port **20** to the first filter **23,** the pair of thin rubber plates of the check valve **27** are deformed so that their interspace expands; this causes the check valve **27** to be open. On the other hand, when air tries to flow from the first filter **23** and the intake port **20,** the pair of thin rubber plates will deform so as to abut with each other; this causes the check valve **27** to be closed.

As shown in FIG. **6****,** when the pressure at the first aperture **24a** side and the pressure at the intake port **20** side are equal, i.e., when there is no air movement in the two aforementioned directions, the leading ends of the pair of thin rubber plates of the check valve **27** are spaced apart. In other words, when there is no air movement, the pair of thin rubber plates do not abut with each other. Because of the check valve **27** having this structure, even when the reference gas generator **17** has sucked in highly humid air, the pair of thin rubber plates are restrained from adhering to each other due to humidity.

The supply tube **29** has a third aperture **29a** and a fourth aperture **29b;** as the attachment section **28** of the case is inserted in the fourth aperture **29b,** the fourth aperture **29b** becomes connected to the intake port **20.** As will be described in detail below, the distance from the third aperture **29a** of the supply tube **29** to the first aperture **24a** of the first filter **23** is longer than the distance between the first aperture **24a** and the second aperture **25a** of the first filter. Moreover, the distance between the third aperture **29a** and the fourth aperture **29b** of the supply tube **29** is longer than the distance between the first aperture **24a** and the second aperture **25a** of the first filter.

Preferably, the distance between the third aperture **29a** of the supply tube **29** and the fourth aperture **29b** is 50 mm or more, and more preferably 100 mm or more.

The channel of the supply tube **29** has a cross section which is smaller than a cross section of the first filter that is perpendicular to a straight line connecting between the first aperture **24a** and the second aperture **25a,** i.e., the longitudinal direction. Preferably, the supply tube **29** has an inner diameter of 5 mm or less.

The supply tube **29** is composed of a resin. Preferably, it is composed of a resin having low gas permeability. For example, the supply tube **29** is preferably composed of a soft urethane, a fluoroplastic such as polytetrafluoroethylene, or the like.

Since the reference gas generator **17** includes the supply tube **29,** even when the exhalation measurement device **1** is stored in an environment containing alcohol or the like, deterioration of the first filter **23** is suppressed. This can prolong the period during which highly accurate measurement is possible, thus being able to realize an exhalation measurement device which is capable of stably detecting the amount of specific gas while maintaining high accuracy.

### (gas transporter 18)

The gas transporter **18** selectively transports the exhalation which is retained in the chamber **8** or the reference gas which is generated by the reference gas generator to the measurement device **12.** In the present embodiment, the gas transporter **18** includes the switching device **9,** the flow rate detector **10,** and the pump **11.**

FIG. **7** is a schematic diagram showing an exemplary structure of the switching device **9.** The switching device **9** switches between the first path **54** that connects to the chamber **8** and the second path **55** that connects to the reference gas generator **17,** and allows it to be connected to the third path **59.** Specifically, the switching device **9** includes a valve **56** that is located between the first path **54** and the third path, a valve **57** that is located between the second path **55** and the third path **59,** and a driving device **58.** Under control of the control circuit **50,** the driving device 58 opens or closes the valve **56** and the valve **57.** As a result, the switching device **9** selectively connects the first path **54** or the second path **55** to the third path. As the pump **11** sucks in the gas in the selected path, the exhalation or the reference gas is selectively transported to the measurement device **12.**

As the switching device **9,** various valves to be used in fluid control can be used, e.g., a three-way valve. Alternatively, a plurality of solenoid valves may be used. Furthermore, the gas transporter **18** may include two parallel paths that respectively connect the chamber **8** and reference gas to the measurement device, such that a valve, a pump, and a flow rate detector are provided in each path. In this case, by selectively operating the valves and pumps in the two paths, the exhalation or the reference gas can be selectively transported to the measurement device **12.** As the pump **11,** commercially available pumps for use with various fluids can be used.

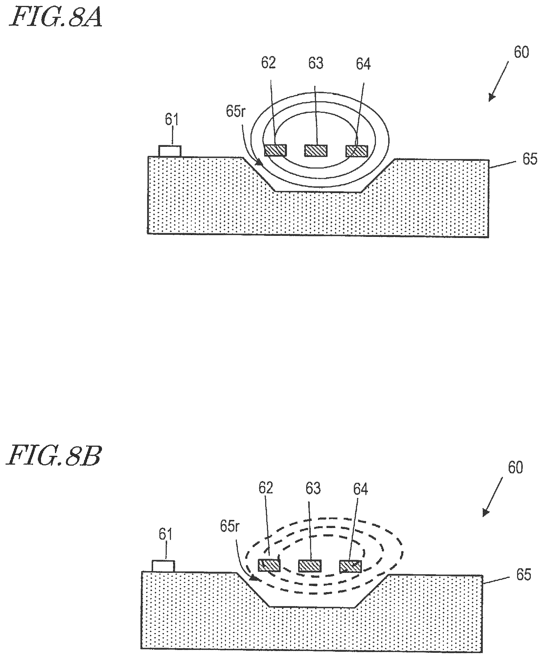

The flow rate detector **10** detects a flow rate of a gas flowing in the third path **59.** As the flow rate detector **10,** various sensors that detect a flow rate of a fluid can be used. For example, a sensor of a thermal type, a differential-pressure type, etc., can be used. FIG. **8A** and FIG. **8B** are schematic diagrams showing a thermal-type flow rate detector **60** for use in the flow rate detector **10.** The thermal-type flow rate detector includes, for example, an upstream temperature sensor **62** a heater **63,** and a downstream temperature sensor **64** that are disposed so as to be spaced apart from the bottom of a recess **65r** which is made in a substrate **65** of silicon. Moreover, an ambient temperature sensor **61** is provided on the substrate **65.** When an electric current is flowed in the heater **63,** with heating of the heater, a temperature distribution that is ascribable to the heating emerges above the recess **65r.** As indicated by solid lines in FIG. **8A****,** when the gaseous body covering the recess **65r** is not moving, this temperature distribution is symmetric, so that the upstream temperature sensor **62** and the downstream temperature sensor **64** will detect an equal temperature. However, in an environment where the surrounding gaseous body is moving, as indicated by broken lines in FIG. **8B****,** the temperature distribution will abound at the downstream side, so that a difference will exist between the temperatures detected by the upstream temperature sensor **62** and the downstream temperature sensor **64.** This temperature difference varies with the velocity with which the fluid moves, i.e., flow rate. The detected flow rate may be corrected based on the temperature in the surroundings as obtained by the ambient temperature sensor **61,** whereby a flow rate that is independent of the ambient temperature can be detected.

Otherwise, as a flow rate detector of the differential-pressure type, a differential-pressure type pressure sensor illustrated in FIG. **5B** may also be used, in which case a flow rate can also be measured, for example.

### (measurement device 12)

The measurement device **12** detects a specific gas that is contained in exhalation. As mentioned above, the specific gas is nitrogen monoxide in the present embodiment. As used herein, "specific" refers to an ability to selectively detect some of the various elements and molecules that are contained within exhalation, without having to detect only one specific species. For example, the measurement device **12** may at least have a sensitivity that is low enough to indicate substantially zero sensitivity for nitrogen, oxygen, water, and carbon dioxide that are contained in large amounts in exhalation, while being able to detect nitrogen monoxide. For example, even if the measurement device **12** is able to detect a molecule other than nitrogen monoxide that is hardly contained in exhalation, it suffices if that molecule is substantially not contained in exhalation.

As the measurement device **12,** for example, sensors based on detection principles such as the electrochemical type, the optical type, the semiconductor type, etc., can be used. Specifically, electrons occurring when nitrogen monoxide molecules that are diffused within the sensor undergo an oxidation-reduction reaction with a catalyst may be detected with electrodes in the sensor, and based on the detected amount of electric current, a concentration of nitrogen monoxide can be measured.

### [operation of the exhalation measurement device 1]

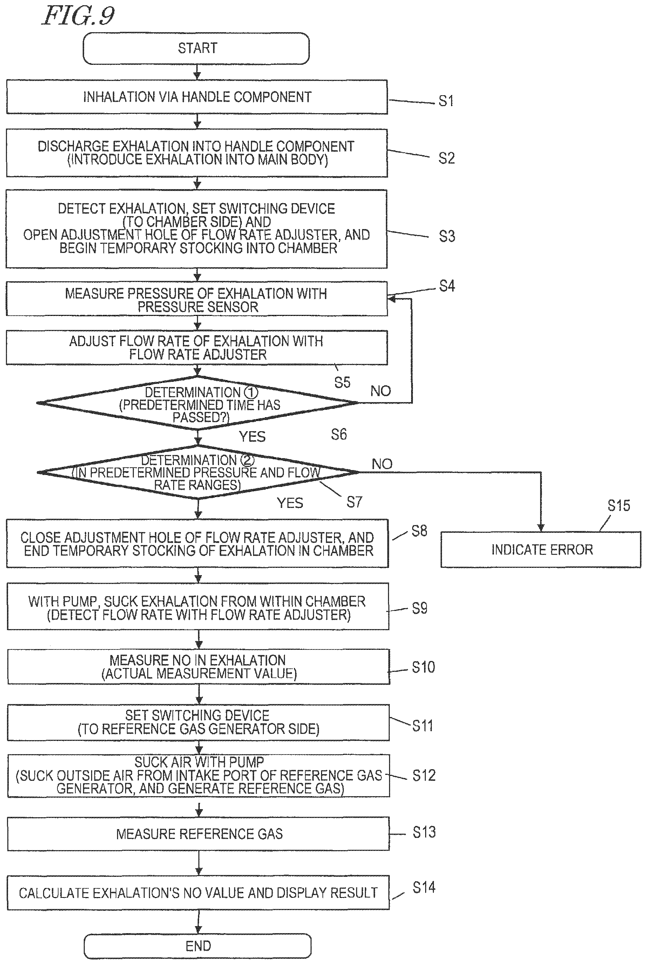

Next, with reference to FIG. **2** and FIG. **9****,** an operation of the exhalation measurement device will be described. FIG. **9** is a flowchart illustrating an operation of the exhalation measurement device. The measurement can be generally classified into: steps of generating exhalation and temporarily stocking the exhalation in the chamber **8** (**S1** to **S8);** and steps of measuring a concentration of the specific gas within the exhalation retained in the chamber **8 (S9** to **S14**).

### (step of stocking exhalation)

First, the subject or operator turns ON the power switch **15** of the exhalation measurement device **1,** and presses a button to begin measurement from the input device **13,** thus beginning measurement. Instead of beginning measurement with an input via the input device **13,** a change in pressure that is associated with the subject generating exhalation may be detected by the pressure sensor **6,** thus beginning measurement. Thereafter, in accordance with the procedure in the flowchart shown in FIG. **9****,** the control circuit **50** controls the respective component elements of the exhalation measurement device **1.** When beginning measurement, the valve **56** provided in the first path **54** of the gas transporter **18** is closed so that exhalation will not move from the chamber **8** toward the pump **11.**

The subject holds the handle section **2** in a hand, places the orifice **5** against his or her mouth, and breathes in. As a result of this, the lungs of the subject are filled with the air around the exhalation measurement device **1,** which has passed through the second filter **34** and from which nitrogen monoxide has been removed (**S1**). Next, the subject breathes out, so as to blow exhalation into the handle section **2** through the aperture. Via the handle tube **3,** the exhalation is introduced into the introduction path **51** of the main body **4** (**S2**).

The pressure sensor **6** detects a change in pressure in the introduction path **51,** and the control circuit **50** moves the adjustment valve **52** of the flow rate adjuster **7.** As a result, the exhalation is transported to the chamber **8** through the introduction path **51** (**S3**). Moreover, the valve **56** provided in the first path **54** of the gas transporter **18** opens.

While the exhalation is being blown in, the pressure of the exhalation is measured by the pressure sensor **6** (**S4**), and based on the result of measurement, the control circuit **50** moves the adjustment valve **52** of the flow rate adjuster **7** in the introduction path **51,** whereby the flow rate of the exhalation moving in the introduction path **51** is adjusted (**S5**).

The control circuit measures an elapsed time since the pressure sensor **6** detected a change in pressure, and pressure measurement (**S4**) and adjustment of the flow rate of the exhalation **(S5)** are performed until a predetermined time elapses (**S6**).

After the lapse of the predetermined time, the control circuit **50** determines whether the pressure is within a predetermined range, and whether the flow rate is within a predetermined range (**S7**). If the pressure and the flow rate are within the predetermined ranges **(S7),** the control circuit **50** closes the adjustment valve **52** of the flow rate adjuster **7,** thus ending stocking of the exhalation in the chamber **8 (S8).** The control circuit **50** causes the display device **14** to display information indicating that exhalation has been properly acquired. With this, the subject ends blowing of breath, and disengages the handle section **2** from his or her mouth.

If the pressure and the flow rate are outside the predetermined ranges **(S7),** the control circuit **50** causes the display device **14** to display information indicating failure of measurement (**S15**). Also, information to prompt another measurement is displayed. With this, the subject or operator carries out another measurement (**S1** to **S8)).**

In the above processes, the air that existed in the introduction path **51** prior to beginning the measurement first flows into the chamber **8.** Thereafter, as the exhalation is introduced into the chamber **8,** the air that first flowed into the chamber **8** is discharged through the discharge port of the chamber **8.** With such operation, in the case where the blowing time of exhalation is set to about 30 seconds, for example, the exhalation which is obtained in the last few seconds in the blowing time of exhalation will be retained in the chamber **8.**

### (measurement step)

The control circuit **50** drives the pump **11** of the gas transporter **18** to transport the exhalation in the chamber **8** to the measurement device **12** (**S9**). At this point, a flow rate is detected by using the flow rate detector **10,** and based on the result of detection, the pump **11** is controlled to attain a predetermined flow rate, thereby adjusting the flow rate of exhalation being introduced into the measurement device **12.** The control circuit **50** drives the pump **11** for a certain period of time. Thus, the measurement device **12** detects the flow rate of nitrogen monoxide in the exhalation which comes transported by the pump **(S10).**

When measurement of a predetermined amount of exhalation is completed, the control circuit **50** controls the switching device **9** of the gas transporter **18** so as to connect the second path **55** that connects to the reference gas generator **17** and the third path **59** that connects to the pump **11** (**S11**), and controls the pump **11** so that the reference gas will be introduced into the measurement device **12** similarly to exhalation. The pump **11** sucks the reference gas that is generated via passage through the reference gas generator **17,** and transports it to the measurement device **12** (**S12**). The measurement device **12** detects a concentration of nitrogen monoxide in the reference gas that comes transported by the pump (**S13**).

After the measurement is completed, the control circuit **50** corrects the result of measurement of concentration of nitrogen monoxide in the exhalation by using a result of measurement of concentration of nitrogen monoxide in the reference gas, and displays it on the display device **14** as the result of measurement (**S14**). Thus, exhalation measurement by the exhalation measurement device **1** is ended. The concentration of nitrogen monoxide within exhalation is, generally speaking, a concentration of several ppb to 50 ppb, for example.

With the exhalation measurement, device **1** according to the present embodiment, since the supply tube **29** is connected to the reference gas generator **17,** even if the exhalation measurement device **1** is stored under varying environments in a medical institution, deterioration in the adsorption ability of the first filter of the reference gas generator **17** is suppressed. This will be described with reference to experimental results.

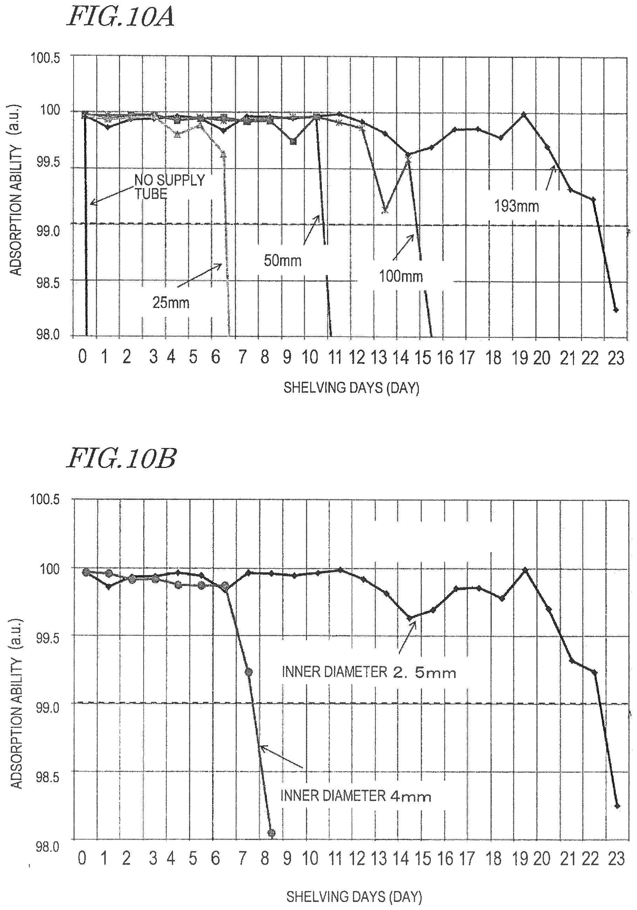

FIG. **10A** shows results of examining deteriorations in performance of the first filter, where polytetrafluoroethylene tubes with an inner diameter of 2.5 mm, having respective lengths of 25 mm, 50 mm, 100 mm, and 193 mm, were prepared and used as the supply tube **29** of the reference gas generator **17.**

The first filter has a diameter of 12, and a length of 32 mm. The reference gas generator **17** was stored in an environment containing 4% alcohol, and its adsorption ability was regularly measured. The horizontal axis represents the number of storing days, and the vertical axis represents an adsorption ability against the nitrogen monoxide adsorption ability of the reference gas generator **17** before storage, which is defined as 100. For comparison, a result of a reference gas generator which was not connected to the supply tube is also shown. FIG. **10B** shows experimental results following a similar procedure, where the supply tubes **29** had respective inner diameters of 4 mm and 2.5 mm. The supply tubes **29** all had a length of 193 mm.

As shown in FIG. **10A**, in the absence of the supply tube **29,** the adsorption ability lowers to 98% or less immediately after start of the experiment. On the other hand, when the supply tube is 25 mm long, an adsorption ability of 99% or more is maintained until 6 days have passed. With the supply tubes **29** that are 50 mm, 100 mm, and 193 mm long, an adsorption ability of 99% or more is maintained up to 10 days, 14 days, and 22 days, respectively. Thus, it is indicated that, as the supply tube becomes longer, the adsorption ability is maintained for longer periods.

Moreover, as shown in FIG. **10B****,** it can be seen that the period during which high adsorption ability can be maintained becomes longer as the inner diameter of the supply tube **29** becomes smaller. In the cases where the inner diameter is 4 mm and 2.5 mm, respectively, an adsorption ability of 99% or more is maintained up to 7 days and 22 days.

The reason why providing the supply tube allows the nitrogen monoxide adsorption ability of the first filter to be maintained for a long period of time is unclear as yet. According to a study by the inventors, alcohol molecules that have arrived at the first filter **23** are adsorbed by the first filter **23,** and therefore, when the supply tube **29** is stored in an alcohol-containing environment, the alcohol concentration near the first aperture **23a** of the first filter **23** is considered to always have a near-zero value. On the other hand, the third aperture **29a** of the supply tube **29** is in contact with the environmental ambient. As a result, the difference in alcohol concentration between the third aperture **29a** of the supply tube **29** and the first aperture **23a** of the first filter **23** is always constant, and alcohol's arrival at the first filter **23** is governed by a diffusion that is based on a concentration gradient, such that the amount of diffused alcohol per unit area remains constant irrespective of lapse of time. Presumably for this reason, the number of alcohol molecules arriving at the first filter **23** decreases as the length of the supply tube **29** becomes longer, and as the cross-sectional area (inner diameter) becomes smaller.

Based on these results, in the exhalation measurement device according to the present embodiment, the performance of the reference gas generator **17** is less likely to deteriorate even in an environment containing alcohol or the like, and the reference gas generator **17** is able to generate for long periods of time a reference gas from which a specific gas has been adequately removed. Thus, a highly accurate measurement is possible by using the reference gas for measuring the specific gas within exhalation, and the highly accurate measurement can be performed stably for long periods of time. Although alcohol is used in the experimental example, the exhalation measurement device according to the present disclosure is considered to provide similar effects also for any compounds other than alcohol that may degrade the adsorption ability of the first filter **23.**

### [other implementations]

The exhalation measurement device according to the present disclosure admits of various modifications. For example, as the supply tube **29** of the reference gas generator, from the standpoint of flexibility for facilitating installation within the space of the main body **4,** a resin supply tube would be suitable. However, when previous designing of the placement of the supply tube **29** within the main body **4** is possible, a hard resin tube, a metal tube, or a tube including overlaid resin moldings, etc., may be used to form the supply tube. If the supply tube is composed of a metal such as iron or copper, any gaseous body that tries to permeate into the supply tube from the metal tubular body can be blocked. In the case of adopting a supply tube made of any such hard material, a construction in which the case **19** of the reference gas generator **17** and the supply tube **29** are integrally formed may be adopted.

Although the embodiment illustrates the exhalation measurement device with respect to an example of detecting nitrogen monoxide within exhalation, the exhalation measurement device may also measure the concentration of any other gas besides nitrogen monoxide. For example, carbon monoxide, aldehydes, or the like may be measured. In this case, the exhalation measurement device would include a measurement device **12** which is suitable for the measurement of carbon monoxide, aldehydes, etc., and a first filter and a second filter containing an adsorbent that adsorbs carbon monoxide, aldehydes, etc.

### INDUSTRIAL APPLICABILITY

The present disclosure can be utilized for exhalation measurement devices to be used in e.g. detecting asthma or checking pulmonary function.

### REFERENCE SIGNS LIST

- **1**: exhalation measurement device
- **2**: handle section
- **3**: handle tube
- **4**: main body
- **5**: orifice
- **6**: pressure sensor
- **7**: flow rate adjuster
- **8**: chamber
- **9**: switching device
- **10**: flow rate detector
- **11**: pump
- **12**: measurement device
- **13**: input device
- **14**: display device
- **15**: power switch
- **16**: memory
- **17**: reference gas generator
- **18**: gas transporter
- **19**: case
- **20**: intake port
- **21**: exhaust port
- **22**: aeration path
- **23**: first filter
- **23a**: first aperture
- **24**: gasket
- **24a**: first aperture
- **25**: gasket
- **25a**: second aperture
- **26**: adsorbent
- **27**: check valve
- **28**: attachment section
- **29**: supply tube
- **29a**: third aperture
- **29b**: fourth aperture
- **31**: sub-housing
- **32**: inhalation path
- **33**: inhalation path
- **34**: second filter
- **34a**: aperture
- **34b**: adsorbent
- **34c**: aperture
- **35A, 35B**: one-way valve
- **36**: suction hole
- **37**: discharge hole
- **41**: main housing
- **41a**: external air hole
- **50**: control circuit
- **51**: introduction path
- **51a**: adjustment hole
- **51b**: port
- **51c**: port
- **52**: adjustment valve
- **52a**: tapered portion
- **53**: driving device
- **54**: first path
- **55**: second path
- **56**: valve
- **57**: valve
- **58**: driving device
- **59**: third path
- **60**: thermal-type flow rate detector
- **61**: ambient temperature sensor
- **62**: upstream temperature sensor
- **63**: heater
- **64**: downstream temperature sensor
- **65**: substrate
- **65r**: recess

## Claims

1. An exhalation measurement device comprising:
a chamber (8) to retain exhalation;
a reference gas generator (17) to generate a reference gas; and
a measurement device (12) to measure concentrations, respectively, of exhalation being retained in the chamber (8) and a specific gas within the reference gas,
the reference gas generator (17) including:
a case (19) having an intake port (20) and an exhaust port (21), and an aeration path (22) connecting the intake port (20) and the exhaust port (21);
a first filter (23) having a first aperture (24a) and a second aperture (25a) respectively on the intake port side and the exhaust port side in the aeration path (22) within the case (19), the first filter (23) being disposed in the aeration path (22) and includes an adsorbent (26) to adsorb the specific gas; and
**characterized by** a supply tube (29) having a third aperture (29a) and a fourth aperture (29b), the fourth aperture (29b) being connected to the intake port (20) of the case (19), wherein
a distance from the third aperture (29a) of the supply tube (29) to the first aperture (24a) of the first filter (23) is longer than a distance between the first aperture (24a) and the second aperture (25a) of the first filter (23).

2. The exhalation measurement device of claim 1, wherein a distance between the third aperture (29a) and the fourth aperture (29b) of the supply tube (29) is longer than the distance between the first aperture (24a) and the second aperture (25a) of the first filter (23).

3. The exhalation measurement device of claim 1, wherein the distance between the third aperture (29a) and the fourth aperture (29b) of the supply tube (29) is 50 mm or more.

4. The exhalation measurement device of claim 1, wherein the distance between the third aperture (29a) and the fourth aperture (29b) of the supply tube (29) is 100 mm or more.

5. The exhalation measurement device of claim 1, wherein a channel of the supply tube (29) has a cross section which is smaller than a cross section of the first aperture (24a) that is perpendicular to a straight line connecting the first filter (23) and the second aperture (25a).

6. The exhalation measurement device of claim 1, wherein the supply tube (29) has an inner diameter of 5 mm or less.

7. The exhalation measurement device of claim 1, wherein the supply tube (29) is composed of a resin.

8. The exhalation measurement device of claim 7, wherein the resin is a fluoroplastic.

9. The exhalation measurement device of claim 1, wherein the reference gas generator (17) includes a check valve (27) disposed between the intake port of the case (19) and the first aperture (24a).

10. The exhalation measurement device of claim 1, comprising:
a gas transporter (18) including a pump (11) to selectively transport the exhalation being retained in the chamber (8) or the reference gas generated by the reference gas generator (17) to the measurement device (12); and
a control circuit (50) to control an operation of the gas transporter (18).

11. The exhalation measurement device of claim 10, further comprising an exhalation generation unit (2) including:
an aperture (5) through which exhalation and inhalation pass,
an inhalation path (32) having a suction hole (36) and connecting the aperture (5) with the suction hole (36),
a second filter (34) to adsorb the specific gas, the second filter (34) being located in the inhalation path (32),
an exhalation path (33) having a discharge hole (37) and connecting the aperture (5) with the discharge hole (37), wherein,
exhalation which is sent from the discharge hole (37) is introduced into the chamber (8).

12. The exhalation measurement device of claim 11, further comprising a main housing (41) which accommodates the chamber (8), the reference gas generator (17), the measurement device (12), the gas transporter (18), and the control circuit (50), wherein the exhalation generation unit (2) is mounted to the main housing (41).

13. The exhalation measurement device of claim 11, further comprising a handle section (2) including a sub-housing (31) which is grippable by a subject, wherein
the exhalation generation unit (2) is accommodated in the sub-housing (31).

14. The exhalation measurement device of claim 10, further comprising:
an introduction path (51) having an inlet through which the exhalation is introduced, the introduction path (51) being connected to the chamber (8);
a flow rate adjuster (7) to adjust a flow rate of the exhalation flowing in the introduction path (51); and
a pressure sensor (6) to measure a pressure in the introduction path (51), wherein,
the control circuit (50) controls the flow rate adjuster (7) based on an output from the pressure sensor (6).

15. The exhalation measurement device of claim 10, wherein,
the gas transporter (18) further includes
a switching device (9) to switch between a first path (54) connected to the chamber (8) and a second path (55) connected to the pump (11), and allow the first path (54) or the second path (55) to connect to a third path (59) that is connected to the pump (11), and
a flow rate detector (10) to measure a flow rate of the exhalation or the reference gas flowing in the third path (59); and
the control circuit (50) controls the switching device (9), and controls the pump (11) based on an output from the flow rate detector (10).

## Patentansprüche

1. Exspirations-Messvorrichtung, die umfasst:
eine Kammer (8) zum Zurückhalten von Exspiration;
eine Referenzgas-Erzeugungseinrichtung (17) zum Erzeugen eines Referenzgases; und
eine Messvorrichtung (12) zum Messen von Konzentrationen in der Kammer (8) zurückgehaltener Exspiration bzw. eines bestimmten Gases in dem Referenzgas,
wobei die Referenzgas-Erzeugungseinrichtung (17) einschließt:
ein Gehäuse (19) mit einem Einlassanschluss (20) und einem Auslassanschluss (21) sowie einem Belüftungsweg (22), der den Einlassanschluss (20) und den Auslassanschluss (21) verbindet;
einen ersten Filter (23) mit einer ersten Öffnung (24a) und einer zweiten Öffnung (25a) an der Seite des Einlassanschlusses bzw. der Seite des Auslassanschlusses auf dem Belüftungsweg (22) innerhalb des Gehäuses (19), wobei der erste Filter (23) auf dem Belüftungsweg (22) angeordnet ist und ein Adsorptionsmittel (26) zum Adsorbieren des bestimmten Gases enthält; und
**gekennzeichnet durch** ein Zuführrohr (29) mit einer dritten Öffnung (29a) und einer vierten Öffnung (29b), wobei die vierte Öffnung (29b) mit dem Einlassanschluss (20) des Gehäuses (19) verbunden ist, und
ein Abstand von der dritten Öffnung (29a) des Zuführrohrs (29) zu der ersten Öffnung (24a) des ersten Filters (23) größer ist als ein Abstand zwischen der ersten Öffnung (24a) und der zweiten Öffnung (25a) des ersten Filters (23).

2. Exspirations-Messvorrichtung nach Anspruch 1, wobei ein Abstand zwischen der dritten Öffnung (29a) und der vierten Öffnung (29b) des Zuführrohrs (29) größer ist als der Abstand zwischen der ersten Öffnung (24a) und der zweiten Öffnung (25a) des ersten Filters (23).

3. Exspirations-Messvorrichtung nach Anspruch 1, wobei der Abstand zwischen der dritten Öffnung (29a) und der vierten Öffnung (29b) des Zuführrohrs (29) 50 mm oder mehr beträgt.

4. Exspirations-Messvorrichtung nach Anspruch 1, wobei der Abstand zwischen der dritten Öffnung (29a) und der vierten Öffnung (29b) des Zuführrohrs (29) 100 mm oder mehr beträgt.

5. Exspirations-Messvorrichtung nach Anspruch 1, wobei ein Kanal des Zuführrohrs (29) einen Querschnitt hat, der kleiner ist als ein Querschnitt der ersten Öffnung (24a), die senkrecht zu einer geraden Linie ist, die den ersten Filter (23) und die zweite Öffnung (25a) verbindet.

6. Exspirations-Messvorrichtung nach Anspruch 1, wobei das Zuführrohr (29) einen Innendurchmesser von 5 mm oder weniger hat.

7. Exspirations-Messvorrichtung nach Anspruch 1, wobei das Zuführrohr (29) aus einem Harz besteht.

8. Exspirations-Messvorrichtung nach Anspruch 7, wobei das Harz ein fluorhaltiger Kunststoff ist.

9. Exspirations-Messvorrichtung nach Anspruch 1, wobei die Referenzgas-Erzeugungseinrichtung (17) ein Rückschlagventil (27) enthält, das zwischen dem Einlassanschluss des Gehäuses (19) und der ersten Öffnung (24a) angeordnet ist.

10. Exspirations-Messvorrichtung nach Anspruch 1, die umfasst:
eine Gas-Transporteinrichtung (18), die eine Pumpe (11) zum selektiven Transportieren der in der Kammer (8) zurückgehaltenen Exspiration oder des von der Referenzgas-Erzeugungseinrichtung (17) erzeugten Referenzgases zu der Messvorrichtung (12) einschließt; sowie
eine Steuerschaltung (50) zum Steuern einer Funktion der Gas-Transporteinrichtung (18).

11. Exspirations-Messvorrichtung nach Anspruch 10, die des Weiteren eine Einheit (2) für Erzeugung von Exspiration umfasst, die einschließt:
eine Öffnung (5), durch die Exspiration und Aspiration hindurchtreten,
einen Aspirations-Weg (32), der ein Ansaugloch (36) aufweist und die Öffnung (5) mit dem Ansaugloch (36) verbindet,
einen zweiten Filter (34) zum Adsorbieren des bestimmten Gases, wobei der zweite Filter (34) sich auf dem Aspirations-Weg (32) befindet,
einen Exspirations-Weg (33), der ein Ausstoßloch (37) aufweist und die Öffnung (5) mit dem Ausstoßloch (37) verbindet, wobei
Exspiration, die über das Ausstoßloch (37) ausgeleitet wird, in die Kammer (8) eingeleitet wird.

12. Exspirations-Messvorrichtung nach Anspruch 11, die des Weiteren ein Hauptgehäuse (41) umfasst, das die Kammer (8), die Referenzgas-Erzeugungseinrichtung (17), die Messvorrichtung (12), die Gas-Transporteinrichtung (18) sowie die Steuerschaltung (50) aufnimmt, wobei
die Einheit (2) für Erzeugung von Exspiration an dem Hauptgehäuse (41) angebracht ist.

13. Exspirations-Messvorrichtung nach Anspruch 11, die des Weiteren einen Griffabschnitt (2) umfasst, der ein Nebengehäuse (31) einschließt, das von einem Patienten ergriffen werden kann, wobei
die Einheit (2) für Erzeugung von Exspiration in dem Nebengehäuse (31) aufgenommen ist.

14. Exspirations-Messvorrichtung nach Anspruch 10, die des Weiteren umfasst:
einen Einleitungs-Weg (51) mit einem Einlass, über den die Exspiration eingeleitet wird, wobei der Einleitungs-Weg (51) mit der Kammer (8) verbunden ist;
eine Strömungsgeschwindigkeits-Reguliereinrichtung (7) zum Regulieren der Strömungsgeschwindigkeit der auf dem Einleitungs-Weg (51) strömenden Exspiration; und
einen Drucksensor (6) zum Messen eines Drucks auf dem Einleitungs-Weg(51), wobei,
die Steuerschaltung (50) die Strömungsgeschwindigkeits-Reguliereinrichtung (7) auf Basis eines Ausgangs von dem Drucksensor (6) steuert.

15. Exspirations-Messvorrichtung nach Anspruch 10, wobei
die Gas-Transporteinrichtung (18) des Weiteren einschließt:
eine Umschaltvorrichtung (9), die zwischen einem ersten Weg (54), der mit der Kammer (8) verbunden ist, und einem zweiten Weg (55) umschaltet, der mit der Pumpe (11) verbunden ist, und Verbinden des ersten Weges (54) oder des zweiten Weges (55) mit einem dritten Weg (59) zulässt, der mit der Pumpe (11) verbunden ist, sowie
eine Strömungsgeschwindigkeits-Erfassungseinrichtung (10) zum Messen einer Strömungsgeschwindigkeit der Exspiration oder des Referenzgases, die/das auf dem dritten Weg (59) strömt; und
die Steuerschaltung (50) die Umschaltvorrichtung (9) steuert und die Pumpe (11) auf Basis eines Ausgangs von der Strömungsgeschwindigkeits-Erfassungseinrichtung (10) steuert.

## Revendications

1. Dispositif de mesure d'expiration comprenant :
une chambre (8) pour retenir l'expiration ;
un générateur de gaz de référence (17) pour générer un gaz de référence ; et
un dispositif de mesure (12) pour mesurer les concentrations, respectivement, de l'expiration qui est retenue dans la chambre (8) et d'un gaz spécifique à l'intérieur du gaz de référence,
le générateur de gaz de référence (17) comprenant :
un logement (19) ayant un orifice d'admission (20) et un orifice d'échappement (21), et un trajet d'aération (22) reliant l'orifice d'admission (20) et l'orifice d'échappement (21) ;
un premier filtre (23) ayant une première ouverture (24a) et une deuxième ouverture (25a) respectivement du côté de l'orifice d'admission et du côté de l'orifice d'échappement dans le trajet d'aération (22) à l'intérieur du logement (19), le premier filtre (23) étant disposé dans le trajet d'aération (22) et comprend un adsorbant (26) pour adsorber le gaz spécifique ; et
**caractérisé par** un tube d'alimentation (29) ayant une troisième ouverture (29a) et une quatrième ouverture (29b), la quatrième ouverture (29b) étant reliée à l'orifice d'admission (20) du logement (19), dans lequel
une distance entre la troisième ouverture (29a) du tube d'alimentation (29) et la première ouverture (24a) du premier filtre (23) est supérieure à une distance entre la première ouverture (24a) et la deuxième ouverture (25a) du premier filtre (23).

2. Dispositif de mesure d'expiration de la revendication 1, dans lequel une distance entre la troisième ouverture (29a) et la quatrième ouverture (29b) du tube d'alimentation (29) est plus longue que la distance entre la première ouverture (24a) et la deuxième ouverture (25a) du premier filtre (23).

3. Dispositif de mesure d'expiration de la revendication 1, dans lequel la distance entre la troisième ouverture (29a) et la quatrième ouverture (29b) du tube d'alimentation (29) est supérieure ou égale à 50 mm.

4. Dispositif de mesure d'expiration de la revendication 1, dans lequel la distance entre la troisième ouverture (29a) et la quatrième ouverture (29b) du tube d'alimentation (29) est supérieure ou égale à 100 mm.

5. Dispositif de mesure d'expiration de la revendication 1, dans lequel un canal du tube d'alimentation (29) a une section transversale qui est inférieure à une section transversale de la première ouverture (24a) qui est perpendiculaire à une ligne droite reliant le premier filtre (23) et la deuxième ouverture (25a).

6. Dispositif de mesure d'expiration de la revendication 1, dans lequel le tube d'alimentation (29) a un diamètre intérieur inférieur ou égal à 5 mm.

7. Dispositif de mesure d'expiration de la revendication 1, dans lequel le tube d'alimentation (29) est composé d'une résine.

8. Dispositif de mesure d'expiration de la revendication 7, dans lequel la résine est un fluoroplastique.

9. Dispositif de mesure d'expiration de la revendication 1, dans lequel le générateur de gaz de référence (17) comprend un clapet anti-retour (27) disposé entre l'orifice d'admission du logement (19) et la première ouverture (24a).

10. Dispositif de mesure d'expiration de la revendication 1, comprenant :
un transporteur de gaz (18) comprenant une pompe (11) pour transporter sélectivement l'expiration qui est retenue dans la chambre (8) ou le gaz de référence généré par le générateur de gaz de référence (17) vers le dispositif de mesure (12) ; et
un circuit de commande (50) pour commander un fonctionnement du transporteur de gaz (18).

11. Dispositif de mesure d'expiration de la revendication 10, comprenant en outre une unité de génération d'expiration (2) comprenant :
une ouverture (5) à travers laquelle passent l'expiration et l'inspiration,
un trajet d'inspiration (32) ayant un trou d'aspiration (36) et reliant l'ouverture (5) au trou d'aspiration (36),
un deuxième filtre (34) pour adsorber le gaz spécifique, le deuxième filtre (34) étant situé dans le trajet d'inspiration (32),
un trajet d'expiration (33) ayant un trou de décharge (37) et reliant l'ouverture (5) au trou de décharge (37), dans lequel,
l'expiration qui est envoyée depuis le trou de décharge (37) est introduite dans la chambre (8).

12. Dispositif de mesure d'expiration de la revendication 11, comprenant en outre un boîtier principal (41) qui loge la chambre (8), le générateur de gaz de référence (17), le dispositif de mesure (12), le transporteur de gaz (18) et le circuit de commande (50), dans lequel
l'unité de génération d'expiration (2) est montée sur le boîtier principal (41).

13. Dispositif de mesure d'expiration de la revendication 11, comprenant en outre une section de poignée (2) comprenant un sous-boîtier (31) qui peut être saisi par un sujet, dans lequel
l'unité de génération d'expiration (2) est logée dans le sous-boîtier (31).

14. Dispositif de mesure d'expiration de la revendication 10, comprenant en outre :
un trajet d'introduction (51) ayant une entrée à travers laquelle l'expiration est introduite, le trajet d'introduction (51) étant relié à la chambre (8) ;
un régulateur de débit (7) pour réguler un débit de l'expiration s'écoulant dans le trajet d'introduction (51) ; et
un capteur de pression (6) pour mesurer une pression dans le trajet d'introduction (51), dans lequel,
le circuit de commande (50) commande le régulateur de débit (7) sur la base d'une sortie du capteur de pression (6).

15. Dispositif de mesure d'expiration de la revendication 10, dans lequel,
le transporteur de gaz (18) comprend en outre
un dispositif de commutation (9) pour commuter entre un premier trajet (54) relié à la chambre (8) et un deuxième trajet (55) relié à la pompe (11), et permettre au premier trajet (54) ou au deuxième trajet (55) d'être relié à un troisième trajet (59) qui est relié à la pompe (11), et
un détecteur de débit (10) pour mesurer un débit de l'expiration ou du gaz de référence s'écoulant dans le troisième trajet (59) ; et
le circuit de commande (50) commande le dispositif de commutation (9) et commande la pompe (11) sur la base d'une sortie du détecteur de débit (10).
